# EUROPEAN PATENT SPECIFICATION

(11) **EP 2 493 522 B1**
(45) Date of publication and mention of the grant of the patent: **16.12.2015**
(21) Application number: 10803485.1
(22) Date of filing: 29.10.2010
(51) Int. Cl.: A61L 27/36, A61L 27/56

(54) **ACELLULAR ORGANIC TISSUE PREPARED FOR REVITALIZATION AND METHOD FOR PRODUCING IT.**
AZELLULÄRES ORGANISCHES GEWEBE ZUR REVITALISIERUNG UND HERSTELLUNGSVERFAHREN DAFÜR
TISSU ORGANIQUE ACELLULAIRE PRÉPARÉ POUR LA REVITALISATION ET SON PROCÉDÉ DE PRODUCTION

(30) Priority: 29.10.2009 IT VI20090263
(43) Date of publication of application: 05.09.2012
(73) Proprietor: Telea Biotech S.r.l., 36066 Sandrigo (VI) (IT)
(72) Inventor: MARZARO, Maurizio, 31100 Treviso (IT)
(74) Representative: Bonini, Ercole
(86) International application number: PCT/IB2010/002767
(87) International publication number: WO 2011/051793

(56) References cited:
- WO-A2-2008/146106
- US-A- 5 112 354
- US-A- 5 658 331
- US-A1- 2004 254 640
- US-A1- 2007 248 638
- CURTIS D CHIN ET AL: "A microfabricated porous collagen-based scaffold as prototype for skin substitutes", BIOMEDICAL MICRODEVICES, KLUWER ACADEMIC PUBLISHERS, BO, vol. 10, no. 3, 23 January 2008 (2008-01-23), pages 459-467, XP019600451,
- TEJAS S KARANDE ET AL: "Diffusion in Musculoskeletal Tissue Engineering Scaffolds: Design Issues Related to Porosity, Permeability, Architecture, and Nutrient Mixing", ANNALS OF BIOMEDICAL ENGINEERING, KLUWER ACADEMIC PUBLISHERS-PLENUM PUBLISHERS, NE, vol. 32, no. 12, 1 December 2004 (2004-12-01), pages 1728-1743, XP019272792, ISSN: 1573-9686
- TIAW K S ET AL: "Laser surface modification of poly(epsilon-caprolactone) (PCL) membrane for tissue engineering applications", BIOMATERIALS, ELSEVIER SCIENCE PUBLISHERS BV., BARKING, GB LNKD- DOI:10.1016/J.BIOMATERIALS.2004.03.010, vol. 26, no. 7, 1 March 2005 (2005-03-01), pages 763-769, XP025280414, ISSN: 0142-9612 [retrieved on 2005-03-01]
- KARANDE TEJAS SHYAM ET AL: "Functions and requirements of synthetic scaffolds in tissue engineering", NANOTECHNOLOGY AND TISSUE ENGINEERING: THE SCAFFOLD, 2008, pages 53-86, XP002585401,
- ISENHATH S N ET AL: "A mouse model to evaluate the interface between skin and a percutaneous device", JOURNAL OF BIOMEDICAL MATERIALS RESEARCH - PART A 20071215 JOHN WILEY AND SONS INC. US LNKD- DOI:10.1002/JBM.A.31391, vol. 83, no. 4, 15 December 2007 (2007-12-15), pages 915-922, XP002585372,

## Description

### Technical background of the invention

The invention concerns a method for the preparation of an acellular organic tissue of human or animal origin for revitalization, in particular for the introduction of living cells, comprising a stage in which the surface of the acellular organic tissue is provided with a plurality of holes extending towards the inside of the tissue, wherein said plurality of holes is made by means of one or more needles.

### State of the art

As is known in the medical sector, and more specifically in the field of surgery, it is increasingly important to be able to use tissues that can be implanted in living beings in order to meet the increasing need to replace parts of organs or whole organs.

The production of biological substitutes that are prepared in laboratories and then implanted in the host, be it an animal or human being, belongs to the medical technique and is known as tissue engineering.

According to a known technique, the preparation of tissues to be transplanted is carried out in the laboratory by inserting cells into a matrix consisting of an inorganic support generally called "scaffold".

The "scaffold", inserted in order to replace a defect in the organ to be treated, favours the three-dimensional organization of the cells until the tissue is completely formed.

Obviously, said scaffold must undergo a degradation process until it disappears completely and is replaced by the regenerated tissue, this process being favoured by the cells grafted in said scaffold.

The transplants obtained with this system can be obtained with both artificial scaffolds and natural scaffolds (from a "donor") that can derive from humans and animals, for example the esophageal wall.

Many publications deal with artificial tissues and among them the following can be mentioned: Tiaw K.S. et al. in "Laser surface modification of poly(epsilon-caprolactone) (PCL) membrane for tissue engineering applications" (Biomaterials 26 (2005) 763-769); Tejas S. Karande et al. in "Diffusion in Musculoskeletal Tissue Engineering Scaffolds: Design Issues Related to Porosity, Permeability, Architecture, and Nutrient Mixing" (Annals of Biomedical Engineering, Vol. 32, no. 12, December 2004, p. 1728-1743); Tejas S. Karande and C. Mauli Agrawal in "Functions and Requirements of Synthetic Scaffolds in Tissue Engineering" ("Nanotechnology and Tissue Engineering: The Scaffolds", 2008 CRC Press, Taylor & Francis Group, 6000 Broken Sound Parkway NW, Suite 300, BOCA RATON, FL 33487-2742 USA, p. 53-86); Curtis D. Chin et al. in "A microfabricated porous collagen-based scaffold as prototype for skin substitutes" (Biomed Microdevices (2008) 10:459-467).

The synthetic tissues described have a structural porosity that, being produced artificially, can be configured in a predetermined way in order to obtain sufficient migration inside the tissue.

Only the last publication mentioned states that cylindrical pores (successively made in the porous tissue) serve to sufficiently improve cell penetration. Scaffolds of human or animal origin already have the natural porosity of the organic tissue, which has an homogeneous aspect resembling the pores of a sponge.

In order to be able to use a scaffold deriving from a donor and then transplant it in a human being, it is necessary to previously treat the tissue in such a way as to completely eliminate all the cells present between the connective tissue fibres and successively reintroduce human cells belonging to the host, so as to avoid rejection reactions.

The techniques for making a scaffold, that is, an acellular matrix starting from tissues taken from a donor, are known and therefore will not be exhaustively described herein, as they substantially consist in the immersion of the tissue to be treated in liquids containing enzymatic substances capable of digesting and destroying the living cells contained in the tissue, while at the same time maintaining the connective tissue fibres intact.

Once an acellular tissue of human or animal origin, ready to receive the cells taken from the host, has been obtained, said tissue or scaffold is placed in a so-called "Petri capsule", a small container used in every biology lab, on the bottom of which the tissue to be revitalized is placed, or in an analogous device.

Revitalization takes place through the introduction of the host's stem cells grown in a culture medium that allows cell nutrition, life, multiplication and diffusion.

Substantially, the stem cells, which at the beginning rest on the upper surface of the tissue, move through the natural interstices of the latter, interstices that were previously occupied by the donor's cells.

After a given lapse of time, with controlled temperatures and in the presence of nutritional substances contained in the culture medium, the living cells are repositioned in the interstices of the tissue which becomes ready to be transplanted in the host's organ.

It can be noticed that generally the cells used for revitalizing the scaffold are stem cells that will differentiate, or have already differentiated, and will assume the specific function of the organ in which the revitalized tissue will be grafted. The success or failure of the transplant of the tissue treated as described depends on the capillary diffusion of the cells inside the tissue.

If said diffusion takes place with difficulty, or superficially and not in depth, the grafted tissue cannot be revitalized and tends to necrotize, which means that the transplant will fail.

The above clearly shows that an essential and important condition to be met, which practically cannot be renounced, is the in-depth revitalization of the tissue in all its parts and above all through its entire depth.

At present, even if the preparation and revitalization treatment takes place for a sufficiently long time, certain results guaranteeing the success of the transplant cannot be obtained.

This is due to the insufficient in-depth penetration of the living cells that must be grafted in the scaffold. Practically, this drawback considerably limits the possibility of preparing tissues suitable for transplants, as rather thick tissues, not being able to be penetrated in depth, are not revitalized completely after the transplant. It is evident, therefore, that the technique described above is suitable only for transplanting tissues with very limited thickness, for example not exceeding approximately 0.1 mm.

In order to overcome this drawback, document WO 2008/146106 A2 filed in the name of the present applicant describes a method according to which the acellular organic tissue is provided with holes in order to favour the diffusion of the cells through all the tissue. Patent US 5,112,354 describes the perforation of a bone part intended to increase the surface exposed to the demineralizing agents and favour the interaction with mesenchymal cells introduced for osteoinduction. US 2007/0248638 A1 describes a method for producing a bioscaffold from natural tissues by oxidizing a decellularized tissue to enlarge the present pores.

According to the mentioned documents, notwithstanding a considerable improvement compared to the traditional techniques, the simple perforation of the tissue with the purpose of preparing it for the introduction of cells still takes a relatively long time to achieve a uniform penetration of the cells, in particular in case of very thick and extended tissues.

Therefore, the natural porosity of the tissue of human or animal origin, together with the holes made as described in document WO 2008/146106 A2, is not optimal yet from the point of view of cell migration inside the tissue.

### Description of the invention

The object of the present invention is to provide a method for producing an acellular organic tissue of human or animal origin ready to be colonized by living cells and capable of overcoming the drawbacks mentioned above.

More specifically, it is the object of the invention to provide a method for producing an acellular organic tissue such that, when said tissue is revitalized with stem cells, compared to the state of the art it is easier for the cells to enter and penetrate every single interstice in the mesh formed by the fibres of the connective tissue, so as to substantially reproduce the conditions of the tissue prior to devitalization.

It is also the object of the invention to obtain a further significant and considerable reduction in the treatment time necessary for revitalizing the acellular scaffold when the living cells intended to prepare the tissue for the transplant are introduced.

Therefore, it is one object of the invention to develop a method for producing an acellular organic tissue prepared in an improved form for the uniform and quick introduction of revitalizing cells.

The objects of the invention are achieved by the method of the type described at the beginning, in which the holes, extending from the outer surface of the tissue towards the inside of the same, intersect at least partially, thus forming holes that are at least partially in communication with each other.

In other words, the tissue that can be obtained with the method of the invention is provided with a plurality of holes, and a single hole communicates along its extension with one or more of the other holes, thus forming a reticular structure. The mesh of communicating holes considerably increases the speed with which they can be filled with regenerating cells. Furthermore, the reticular structure favours the uniform diffusion of the cells.

In the simplest case, said communicating holes are substantially straight, being made with needles through the outer surface of said tissue, so that the holes intersect. The use of curved needles can also be taken in consideration.

None of the documents mentioned in the state of the art discloses interconnected holes added to holes belonging to the structural porosity of the tissue. Rather, the documents that deal with artificial tissues consider the structural porosity (and in one case only, the article of Curtis D. Chin, the application of further cylindrical holes insulated from each other) to be satisfying in terms of the degree of cell penetration that can be obtained.

The holes provided according to the invention are made in such a way that their depth extends at least partially into the thickness of the tissue, but preferably into almost the full thickness of the tissue or, in particular cases, into the full thickness of the tissue.

A thin non perforated layer is preferably left on the side opposite the perforated surface in order to prevent the introduced cells from flowing out. Advantageously, the holes extend uniformly through the whole tissue and along all its sides.

The holes can be made through the thickness of the tissue to be treated with needles and various methods, provided that the execution of said holes does not cause any deterioration or alteration (tears, necrosis, decrease or increase in thickness, alteration of the liquid content, coagulation) of the connective tissue around the hole and in any case the scaffold in general.

According to a particularly advantageous embodiment of the invention, the tissue is also provided with reservoir cavities communicating with at least part of said holes. The reservoir cavities can be considered as recesses, that is, as deposits for reserves of cells to be introduced in the tissue, comparable to deposits of reserve cells in the body (for example, deposits for epithelial cells of the intestine).

If the cells to be introduced in the tissue are no more simply applied to the surface of the tissue, but also introduced in the reservoir cavities made in the tissue that communicate with at least part of the holes provided, it is possible to further improve the uniformity of distribution of the cells and to further accelerate the penetration of the cells throughout the tissue.

The creation of reservoir cavities communicating with at least part of said holes increases, for the cells applied to the tissue (and introduced in the reservoir cavities), the number of access points to the mesh of holes.

Advantageously, the reservoir cavities are on the surface of the tissue in order to make it easier to fill them with cells, that is, to ensure easy access to them. The distribution of the reservoir cavities is preferably uniform.

The diameter of the holes produced, in any case, must be sufficient to allow the cells to easily get into said holes and revitalize the surrounding tissue. Regarding the size of the cells, their diameter should be at least 50 µm.

The selection of the diameter must also be made according to the need to maintain the structural integrity of the tissue, at the same time ensuring quick penetration and complete filling of the holes.

The reservoir cavities can be prepared before or after making the holes.

The channels are preferably applied after the creation of the reservoir cavities, so as to ensure the stability of the tissue.

The reservoir cavities are substantially cylindrical in shape, with diameter up to approximately 1 mm. Reservoir cavities with diameters from 700 µm to 1 mm are particularly suitable for this purpose. The substantially cylindrical reservoir cavities can be easily created as holes made with a corresponding needle having a suitable diameter. Obviously, the diameter of the reservoir cavities must not be such as to weaken the structure of the tissue. The diameter of the reservoir cavities should be larger than the diameter of the channels communicating with them, so that the reservoir cavities can serve as cell reserves and that the channels are not transformed in further reservoir cavities that weaken the structure of the tissue itself. The depth of the reservoir cavities depends on the characteristics of the tissue and can be easily optimized according to the needs by a person skilled in the art.

The same applies to the distance between channels and reservoir cavities. Even distances of approximately 200 µm between the single reservoir cavities can be envisaged, as well as longer or shorter distances.

The ideal distances must be selected according to the needs in terms of penetration speed and stability of the tissue.

In another preferred embodiment of the invention the holes have diameters different from one another. The same applies to the reservoir cavities, which can even have different diameters inside the tissue. The choice of the distribution of the diameters must be determined by a compromise between the increase in penetration speed, which certainly increases as the diameter increases, and the need to avoid weakening the structure of the tissue too much through excessive perforation. The possibility to vary the diameters of the holes inside the tissue offers higher flexibility in the adjustment of the balance between accelerated penetration and stability of the tissue.

In order to efficiently avoid necrosis problems during the formation of the holes and/or reservoir cavities, said plurality of holes and/or reservoir cavities is preferably made by means of one or more metal needles connected to an electric power source, causing on the tip of each needle the passage of an electric current whose intensity and wave shape are such as to supply a quantity of energy sufficient to open the bonds that join the molecules of the organic tissue in proximity to the tip of said needle, as described for example in the already mentioned document WO 2008/146106 A2.

In this way, each hole is such that the tip of said needle enters the space left free by the opening of said molecular bonds and makes said hole. It should be understood that the holes and/or the reservoir cavities can be made also in another manner, for example mechanically.

The best results in terms of quality of the holes/reservoir cavities made in the tissue are obtained by applying to the tip of each needle a high frequency voltage, generally 4 MHz, producing a passage of electric current that is weak but sufficient to open the bonds between the molecules of the connective tissue, so as to make a hole without breaking down the molecules themselves. The holes and/or reservoir cavities are preferably made using a needle powered with a voltage having the wave frequency of 4 MHz indicated above. The voltage is preferably 200-230 Volts. Advantageously, the sinusoidal voltage wave applied is a distorted sinusoidal wave, thus with harmonics at least of the first, second and third order.

The current available on the tip of each needle is preferably adjusted so as to be included between 2 and 2,5 mA.

It is logical and evident that making several intersecting holes means creating new paths for grafting cells down to the deep parts of the tissue, which guarantees complete revitalization of the tissue itself.

The preparation of the acellular organic tissue for revitalization may comprise a step for the introduction of various materials, for example washing solutions, nutrition solutions, cells of different types (for example, autologous cells, from a different donor or of a different type, cells to be differentiated or already differentiated) ecc. Advantageously, the introduction of these materials in the holes takes place with the aid of a suction system that comprises a drive mechanism suited to facilitate the entrance of the materials in the holes.

The suction system may consist of a peristaltic pump. The tissue prepared for revitalization according to the invention is like a permeable sponge for liquids and along the holes also for cells. In the case where the tissue is provided with through holes and therefore does not have a non-perforated layer on the side opposite the perforated surface, the tissue is arranged on a biodegradable membrane provided with micro pores permeable to liquids but not to cells, thus preventing the cells from flowing out during the suction stage. According to the invention, the holes are made in the tissue using a needle or an array of needles moved and inclined according to a control system managed by an algorithm that guarantees that the holes are formed so as to intersect with a predetermined density, that is, forming a determined number of points where they cross each other.

The method described above can be used for all types of organic tissues. The acellular tissues provided with communicating holes can be revitalized with all the types of cells suitable for the future use of the tissue.

The tissue of the invention practically poses no limits to the thickness of the tissue to be transplanted, as the presence of communicating holes that can be made through the full thickness of the tissue and over its entire surface guarantees complete revitalization. This is due to the fact that the living cells that will be reintroduced in the acellular scaffold, and preferably in the reservoir cavities, can reach every single part of the tissue.

The method according to the invention provides an acellular organic tissue of human or animal origin prepared for revitalization, in particular for the introduction of living cells, which comprises holes that are punctures made with needles and extending from the surface of the tissue towards its inside, wherein the holes intersect at least partially and are therefore at least partially communicating with each other.

According to the variants of the method described above the tissue may also comprise reservoir cavities. The holes and/or reservoir cavities in the tissue prepared according to the invention have the characteristics and in particular the diameters, depths and distances described above.

Construction variants of the invention are the subject of the dependent claims. Further characteristics and details of the invention will be highlighted in greater detail in the description of preferred embodiments of the invention provided as non-limiting examples.

### Brief description of the drawings

The invention is now illustrated with the aid of the attached drawings, wherein:
- Figure 1 shows a schematic cross-section of the acellular organic tissue prepared for revitalization and provided with communicating holes;
- Figure 2 shows a top view of the acellular organic tissue prepared for revitalization and provided with reservoir cavities and holes;
- Figure 3 shows a cross-section of the tissue of Figure 2 along line III-III of Figure 2.

### Description of the examples of embodiment

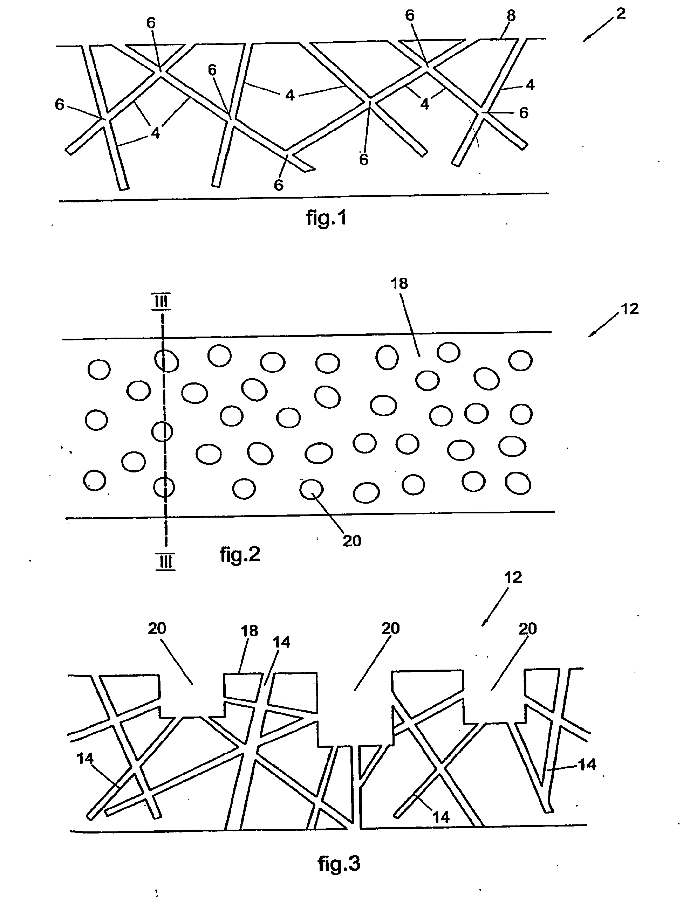

As shown in Figure 1, an acellular organic tissue indicated as a whole by **2** is provided with a plurality of holes **4** that intersect in the crossing points **6** and are therefore communicating with each other.

In the example shown all the holes **4** are inclined with respect to the surface **8** of the tissue **2.** However, it is possible to assume that part of the holes is perpendicular to the surface **8** of the tissue **2.**

Obviously, the penetration/perforation must be repeated over the entire surface **8** of the scaffold **2** in order to obtain an homogeneous distribution of the holes **4** in the entire thickness and over the entire useful surface of the tissue **2** to be grafted.

Figure 2 shows a top view of a further embodiment of the invention, in which an acellular organic tissue **12** is provided not only with holes **14** (for clarity reasons the holes are shown only in the cross section of Figure 2, that is, in Figure 3) but also with reservoir cavities **20.**

It is possible to observe a plurality of reservoir cavities **20** in the form of large holes with circular cross-section on the surface **18** of the tissue **12**.

Finally, Figure 3 shows in a cross-section along line III-III of Figure 2 how the holes **14** communicate with each other and with the reservoir cavities **20**.

Once the series of holes **4, 14** (and if necessary of reservoir cavities **20**) has been made in the acellular tissue **2, 12**, as explained above, it is clear that said tissue without cells can be arranged on a Petri capsule or an analogous device, in which the living cells, generally of the stem type, of the future host are now introduced.

Said stem cells, properly nourished in a culture medium, can easily and quickly get into all the holes **4, 14** provided, thus guaranteeing a complete and effective revitalization of the whole tissue to be transplanted.

A typical process is structured as follows: an organic tissue **2, 12** without cells and thus previously treated, called scaffold, is preferably laid and spread over a plane surface. A needle is powered with electric current so as to supply to the molecules of the tissue to be treated with a quantity of energy that is just sufficient to break the bonds between the molecules involved in the passage of current, while the surrounding area is not affected by any tearing or breaking effect, necrosis, decrease or increase in thickness, or alteration of the liquid content, coagulation or other degenerative effect

Substantially, this opening of the molecular bonds is equivalent to the production of a micro hole **4, 14,** that in practice has the same diameter as each needle, considering, in any case, that the minimum diameter of the needle cannot be less than that of the revitalization cells.

The needle is thus introduced slowly enough and so that during its advance its tip finds the hole already prepared by the passage of current and by the consequent breakage of the molecular bonds.

The reservoir cavities **20** can be produced in the same way, by choosing a needle with suitable diameter, being it preferable but not strictly necessary to make the reservoir cavities before the holes.

The formation of a mesh of holes is particularly important and useful as the cells that will be reintroduced can penetrate the tissue in depth and take root in the walls of the hole, multiply and thus revitalize very quickly all the organic tissue.

It can be understood that the method and the tissue of the invention achieve all the objects of the invention, as perfect and effective revitalization is obtained, thus eliminating any risk of failure of the transplant that must be carried out. Furthermore, the revitalization process takes place even quicker and with a more uniform cell diffusion than in the known technique.

Upon implementation, further changes or construction variants of the method of the invention, not described herein, may be carried out.

Said changes or construction variants must all be considered protected by the present patent, provided that they fall within the scope of the claims expressed below.

Where technical features mentioned in any claim are followed by reference signs, those reference sings have been included for the sole purpose of increasing the intelligibility of the claims and accordingly such reference signs do not have any limiting effect on the protection of each element identified by way of example by such reference signs.

## Claims

1. Method for preparing an acellular organic tissue of human or animal origin for revitalization, in particular for the introduction of living cells, comprising a step in which said acellular organic tissue (2, fig. 1; 12, figs. 2 and 3) is provided with a plurality of holes (4, fig. 1; 14, fig. 3) made through its surface (8, fig. 1; 18, figs. 2 and 3) and extending towards the inside of the tissue (2, fig. 1; 12; figs. 2 and 3), wherein said plurality of holes (4, fig. 1; 14, fig. 3) is made using one or more needles, **characterized in that** said holes (4, fig. 1; 14, fig. 3) intersect at least partially thus forming holes (4, fig. 1; 14, fig. 3) that communicate at least partially with each other by making the holes in the tissue using said needle or an array of needles moved and inclined according to a control system managed by an algorithm that guarantees that the holes are formed so as to intersect with a predetermined density, that is, forming a determined number of points where they cross each other.

2. Method according to claim 1), **characterized in that** it also comprises a step in which reservoir cavities (20, figs. 2 and 3) that communicate with at least part of said holes (14, fig. 3) are created in said tissue (12, figs. 2 and 3).

3. Method according to claim 1) or 2), **characterized in that** said holes and/or reservoir cavities are made by means of one or more metal needles connected to an electric power source, causing on the tip of each needle the passage of a current whose intensity and wave shape are such as to supply a quantity of energy sufficient to open the bonds that join the molecules of the organic tissue in proximity to the tip of said needle.

4. Method according to claim 3), **characterized in that** the electric current is determined by a voltage with a wave frequency of 4 MHz in which the sinusoidal voltage wave applied is a distorted sinusoidal wave and therefore with harmonics at least in the first, second and third order.

5. Method according to any of the preceding claims, **characterized in that** said holes (4, fig. 1; 14, fig. 3) have a diameter of at least 50 µm.

6. Method according to any of the claims from 2) to 5), **characterized in that** said reservoir cavities (20, figs. 2 and 3) are substantially cylindrical and have a diameter of up to 1 mm.

7. Method according to any of the preceding claims, **characterized in that** said holes (4, fig. 1; 14, fig. 3) have different diameters.

8. Method according to any of the claims from 2) to 7), **characterized in that** said reservoir cavities (20, fig. 2 and 3) have different diameters.

9. Method according to any of the preceding claims, **characterized in that** it comprises a step in which various materials are introduced in the holes (4, fig. 1; 14, fig. 3) with the aid of a suction system, wherein the materials are preferably selected among washing solutions, nutrition solutions and/or living cells.

## Patentansprüche

1. Methode zur Herstellung eines azellulären, organischen Gewebes menschlichen oder tierischen Ursprungs zur Revitalisierung, insbesondere zur Besiedelung mit lebenden Zellen, einen Schritt enthaltend, in dem das besagte azelluläre, organische Gewebe (2, Fig. 1; 12, Fig. 2 und 3) mit einer Vielzahl von Löchern (4, Fig. 1; 14, Fig. 3) versehen ist, die durch seine Oberfläche (8, Fig. 1; 18, Fig. 2 und 3) ausgeführt sind und sich in das Innere des Gewebes (2, Fig. 1; 12, Fig. 2 und 3) ausdehnen, wobei die besagte Vielzahl von Löchern (4, Fig. 1; 14, Fig. 3) unter Verwendung von einer oder mehreren Nadeln ausgeführt ist, **dadurch gekennzeichnet, dass** die besagten Löcher (4, Fig. 1; 14, Fig. 3) sich wenigstens teilweise überschneiden und so Löcher (4, Fig. 1; 14, Fig. 3) bilden, die wenigstens teilweise miteinander kommunizieren unter Bildung der Löcher im Gewebe mittels der besagten Nadel oder mit einer Gruppe aus Nadeln, die gemäß eines durch einen Algorithmus gesteuerten Kontrollsystems bewegt und geneigt werden, das sicherstellt, dass die Löcher so gebildet werden, dass sie sich mit einer vorbestimmten Dichte überschneiden, das heißt, unter Bildung einer bestimmten Anzahl von Punkten, in denen sie sich kreuzen.

2. Methode gemäß Patentanspruch 1), **dadurch gekennzeichnet, dass** sie außerdem einen Schritt umfasst, in dem mit wenigstens einem Teil der besagten Löcher (14, Fig. 3) kommunizierende Reservoir-Vertiefungen (20, Fig. 2 und 3) in dem besagten Gewebe (12, Fig. 2 und 3) gebildet werden.

3. Methode gemäß Patentanspruch 1) oder 2), **dadurch gekennzeichnet, dass** die besagten Löcher und/oder Reservoir-Vertiefungen mittels einer oder mehrerer Metallnadeln erzeugt werden, die an eine Stromquelle angeschlossen sind, welche an der Spitze jeder Nadel den Durchfluss von Strom bewirkt, dessen Stärke und Wellenform so beschaffen sind, dass die gelieferte Energiemenge ausreichend ist, um die Bindungen zu öffnen, welche die Moleküle des organischen Gewebes in der Nähe der Spitze der besagten Nadel verbinden.

4. Methode gemäß Patentanspruch 3), **dadurch gekennzeichnet, dass** der elektrische Strom durch eine Spannung mit einer Wellenfrequenz von 4 MHz definiert ist, in der die angewendete Sinusspannungswelle eine verzerrte Sinuswelle ist und somit Harmonische von wenigstens der ersten, zweiten und dritten Ordnung aufweist.

5. Methode gemäß eines jeden der vorstehenden Patentansprüche, **dadurch gekennzeichnet, dass** die besagten Löcher (4, Fig. 1; 14, Fig. 3) einen Durchmesser von wenigstens 50 µm aufweisen.

6. Methode gemäß eines jeden der Patentansprüche von 2) bis 5), **dadurch gekennzeichnet, dass** die besagten Reservoir-Vertiefungen (20, Fig. 2 und 3) im Wesentlichen zylindrisch sind und einen Durchmesser von bis zu 1 mm haben.

7. Methode gemäß eines jeden der vorstehenden Patentansprüche, **dadurch gekennzeichnet, dass** die besagten Löcher (4, Fig. 1; 14, Fig. 3) unterschiedliche Durchmesser aufweisen.

8. Methode gemäß eines jeden der Patentansprüche von 2) bis 7), **dadurch gekennzeichnet, dass** die besagten Reservoir-Vertiefungen (20, Fig. 2 und 3) unterschiedliche Durchmesser haben.

9. Methode gemäß eines jeden der vorstehenden Patentansprüche, **dadurch gekennzeichnet, dass** sie einen Schritt umfasst, in dem mittels eines Ansaugsystems verschiedene Materialien in die Löcher (4, Fig. 1; 14, Fig. 3) gefüllt werden, wobei die Materialien vorzugsweise unter Spül- oder Nährlösungen und/oder lebenden Zellen ausgewählt werden.

## Revendications

1. Méthode de préparation d'un tissu organique acellulaire d'origine humaine ou animale pour la revitalisation, particulièrement pour l'introduction de cellules vivantes, comprenant la phase dans laquelle ledit tissu organique acellulaire (2, Fig. 1; 12, Fig. 2 et 3) est doté d'une pluralité de trous (4, Fig. 1; 14, Fig. 3) réalisés à travers sa surface (8, Fig. 1; 18, Fig. 2 et 3) et s'étendant vers l'intérieur du tissu (2, Fig. 1; 12, Fig. 2 et 3), où ladite pluralité de trous (4, Fig. 1; 14, Fig. 3) est réalisée en utilisant une ou plusieurs aiguilles, **caractérisée en ce que** lesdits trous (4, Fig. 1; 14, Fig. 3) se croisent au moins partiellement en formant de cette façon des trous (4, Fig. 1; 14, Fig. 3) qui communiquent au moins partiellement entre eux en réalisant les trous dans le tissu grâce à l'emploi de ladite aiguille ou une gamme d'aiguilles déplacées et inclinées selon un système de contrôle géré par un algorithme qui garantit que les trous sont formés de façon à se croiser avec une densité prédéterminée, c'est-à-dire, en formant un nombre déterminé de points où ils s'entrecoupent.

2. Méthode selon la revendication 1), **caractérisée en ce qu'**elle comprend en plus une phase d'exécution dans ledit tissu (12, Fig. 2 et 3) de cavités-réservoirs (20, Fig. 2 et 3) qui communiquent avec au moins une partie desdits trous (14, Fig. 3).

3. Méthode selon la revendication 1) ou 2), **caractérisée en ce que** lesdits trous et/ou cavités-réservoirs sont réalisés au moyen d'une ou plusieurs aiguilles métalliques reliées à une source d'alimentation électrique, en causant sur la pointe de chaque aiguille le passage d'un courant dont l'intensité et la forme d'onde sont telles à fournir une quantité d'énergie suffisante à ouvrir les liens qui unissent les molécules du tissu organique à proximité de la pointe de ladite aiguille.

4. Méthode selon la revendication 3), **caractérisée en ce que** le courant électrique est déterminé par une tension avec une fréquence d'onde de 4 MHz où l'onde sinusoïdale de tension appliquée est une onde de type sinusoïdale déformée et donc avec des harmoniques au moins du premier, deuxième et troisième ordres.

5. Méthode selon l'une quelconque des revendications précédentes, **caractérisée en ce que** lesdits trous (4, Fig. 1; 14, Fig. 3) ont un diamètre d'au moins 50 µm.

6. Méthode selon l'une quelconque des revendications de 2) à 5), **caractérisée en ce que** lesdites cavités-réservoirs (20, Fig. 2 et 3) sont essentiellement cylindriques et ont un diamètre jusqu'à 1 mm.

7. Méthode selon l'une quelconque des revendications précédentes, **caractérisée en ce que** lesdits trous (4, Fig. 1; 14, Fig. 3) ont des diamètres différents.

8. Méthode selon l'une quelconque des revendications de 2) à 7), **caractérisée en ce que** lesdites cavités-réservoirs (20, Fig. 2 et 3) ont des diamètres différents.

9. Méthode selon l'une quelconque des revendications précédentes, **caractérisée en ce qu'**elle comprend une phase d'introduction de différents matériaux dans les trous (4, Fig. 1; 14, Fig. 3) à l'aide d'un système d'aspiration, où les matériaux sont préférablement choisis parmi des solutions de lavage, solutions de nourriture et/ou des cellules vivantes.
